# EUROPEAN PATENT SPECIFICATION

(11) **EP 0 979 662 B1**
(45) Date of publication and mention of the grant of the patent: **28.02.2007**
(21) Application number: 99115901.3
(22) Date of filing: 12.08.1999
(51) Int. Cl.: A61M 16/06, A61M 16/08, A61M 16/04

(54) **Mouthpiece, for delivering pressurized gases to a user**
Mundstück zur Verabreichung von Druckgasen an einen Verbraucher
Embouchure pour délivrer des gaz sous pression à un utilisateur

(30) Priority: 13.08.1998 NZ 33135598
(43) Date of publication of application: 16.02.2000
(73) Proprietor: Fisher & Paykel Healthcare Limited, East Tamaki, Auckland (NZ)
(72) Inventor: Gradon, Lewis George, Howick, Auckland (NZ); Smith, Nicholas Charles Alan, Auckland (NZ); Robertson, Christopher John, Dunedin (NZ)
(74) Representative: Hoarton, Lloyd Douglas Charles

(56) References cited:
- EP-A- 0 845 277
- WO-A-90/03199
- WO-A-96/03173
- US-A- 3 139 088
- US-A- 3 670 726
- US-A- 4 270 531
- US-A- 5 333 608

## Description

### BACKGROUND OF THE INVENTION

This invention relates to a mouthpiece for the oral delivery of air in continuous positive airway pressure (CPAP) treatments of sleeping disorders such as sleep apnea.

### PRIOR ART

Sleep apnea treatments have been significantly advanced with the introduction of continuous positive airway pressure (CPAP) treatments. These treatments, as introduced, involve the supply of gases from a gases supply or blower to a patient through a conduit and nasal mask to provide an elevated internal pressure in the users airways to assist the muscles to keep the airways open. This airstream is provided to the user through a nasal mask applied over the nose and held in place by a harness. This configuration has been almost universally adopted based on the well known observation that humans show a decided preference for nasal breathing during sleep. For this reason, little development has been undertaken into other possible methods of providing the pressurized airstream to a user.

Oral delivery is suggested in EP 818, 213, which shows an apparatus for oral delivery of air in a CPAP treatment. The apparatus includes a mouthpiece adapted to fit inside the mouth between the roof of the mouth, the hard palate, and the tongue, and having a periphery which can be gripped between the teeth. It is thought by the applicants that this is significantly more intrusive than is necessary and is liable to movement and consequent discomfort (although not outright removal) under the relaxation of sleep. It has the additional disadvantage that with the user fully relaxed, such as in the case of sleep, a distension in the user's jaw and subsequent opening of the mouth can reduce the sealing effectiveness of the mouthpiece and reduce the efficacy of the CPAP treatment.

Because the mouthpiece in EP 818,213 is gripped between the user's teeth, a further disadvantage results in that the mouthpiece requires custom orthodontic fitting to ensure that the mouthpiece matches the user's mouth and teeth layout. Custom orthodontic fitting is time consuming and removes the capability of effective mass manufacture. Consequently, the mouthpiece in EP 818,213 is expensive, creating a significant barrier to the patient adoption of the device.

A similar gases delivery mouthpiece, for use with a respirator, is shown in WO 90/03199. WO 90/03199 discloses an orthodontic device which is adapted to be gripped between the jaws of a user and to accommodate the user's teeth within a series of upper and lower cavities. A base member of the mouthpiece is shaped and fits against the hard palate of the user. This mouthpiece again has the disadvantage of requiring custom orthodontic fitting. Furthermore, as a result of the mouthpiece's substantial thickness and size, the mouthpiece is substantially rigid in the vestibule regions of the mouth. The mouthpiece is clamped in place by an outer shield which engages the outside of the user's lips.

A paper by E Veres entitled "Clinical trial of an oral vestibular shield for the control of snoring" (Journal of the Dental Association of South Africa, January 1993) describes the use of a shield intended to be retained in the vestibule of the mouth to seal the mouth and to promote nasal breathing which has been conventionally considered to be more beneficial than oral breathing. Humidified CPAP treatments delivered orally, however, actually derive greater benefit than those delivered nasally because secondary leakage through the nasal passages during oral delivery is significantly less than oral leakage during nasal delivery. The shield depicted in the paper is formed from flexible ethylene vinyl. The shield is custom trimmed and is custom fitted by heating to a malleable temperature and deformed by applied pressure.

Other possible mouthpiece designs are shown for example by use in self contained underwater breathing apparatus systems, for example as depicted in United States Patent No. 4,862,909. This mouthpiece is a mouth guard type and is clamped between the teeth. A flange extends both in front of and behind the teeth.

EP-A2-0 845 277 shows the features of the preamble of claim 1.

Prior art mouthpieces are not well adapted for use in CPAP treatments because they are intended for conscious gripping by the user, and have been found subject to accidental removal with a user in a completely relaxed state such as sleep. The present invention overcomes this problem and present several other advantages which will become apparent upon a reading of the attached specification, in combination with a study of the drawings.

### OBJECTS AND SUMMARY OF THE INVENTION

It is an object of the present invention to provide a mouthpiece for oral delivery of gases, which goes some way toward overcoming the above disadvantages or which will at least provide the public with a useful choice.

The invention consists in a mouthpiece as defined in claim 1 with a generally rectangularly-shaped vestibular shield having an inner surface and an outer surface, said vestibular shield having a predetermined height which will overlap a user's teeth and gums when positioned in the mouth vestibule of a user, said vestibular shield having a central portion which will extend over a user's front teeth and gums when said central portion of said vestibular shield is positioned between the lips and the teeth of the user, and outer portions extending from said central portion which extend along and overlap at least a portion of the user's back teeth and gums when said outer portions of said vestibular shield are positioned between the cheeks and the teeth of the user; and gases passageway means extending from said outer surface of said vestibular shield to said inner surface of said vestibular shield for allowing the passage of said gases through said mouthpiece.

### BRIEF DESCRIPTION OF THE DRAWINGS

The organisation and manner of the structure and operation of the invention, together with further objects and advantages thereof, may best be understood by reference to the following description, taken in connection with the accompanying drawings, wherein like reference numerals identify like elements in which:
Figure 1 is a perspective view of a mouthpiece which incorporates features of the present invention as viewed from an outward direction,
Figure 2 is a perspective view of the mouthpiece as viewed from an inward direction and with the mouthpiece upside down,
Figure 3 is a similar perspective view of the mouthpiece, but viewed from further below to show more clearly the form of a lower teeth retaining ridge and the profile of the vestibular shield,
Figure 4 is a side elevational view of the system as being used by a patient,
Figure 5 is a cross sectional view of the mouthpiece and a user with the mouthpiece in place to demonstrate the location and positioning thereof in relation to the main features of a users anatomy,
Figure 6 is a perspective view of a second embodiment of the mouthpiece as viewed from an outward direction,
Figure 7 is a cross section of the mouthpiece of Figure 6,
Figure 8 is a perspective view from above of a mouthpiece according to a further and preferred embodiment of the present invention,
Figure 9 is a perspective view from one side and from an inward direction of the mouthpiece of Figure 8,
Figure 10 is a cross-section of the mouthpiece of Figure 8, and
Figure 11 is a cross-sectional view of the mouthpiece of Figure 8 and a user with the mouthpiece in place to demonstrate the location and positioning thereof in relation to the main features of the user's anatomy.

### DETAILED DESCRIPTION OF THE ILLUSTRATED EMBODIMENT

While the invention may be susceptible to embodiment in different forms, there is shown in the drawings, and herein will be described in detail, specific embodiments with the understanding that the present disclosure is to be considered an exemplification of the principles of the invention, and is not intended to limit the invention to that as illustrated and described herein.

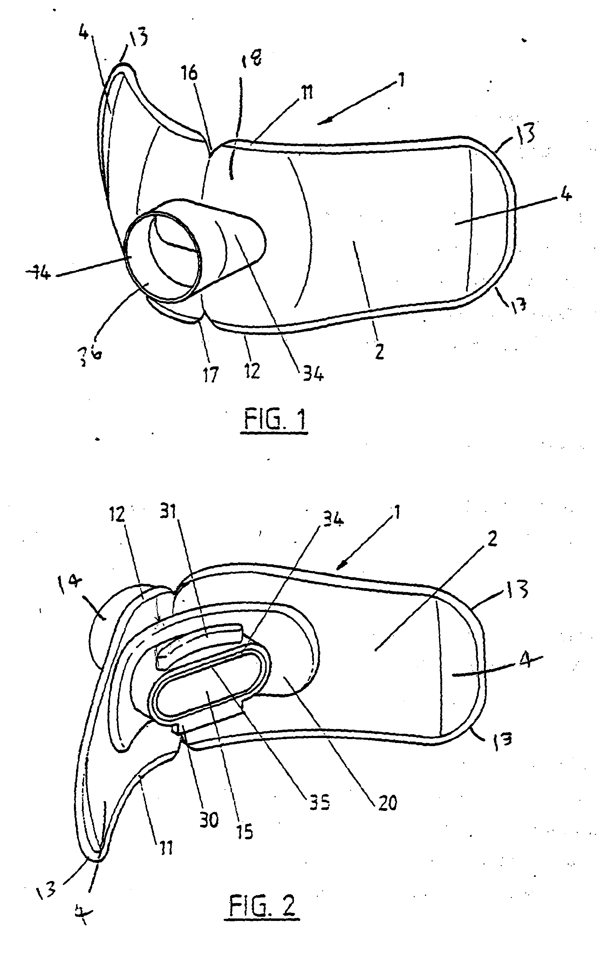

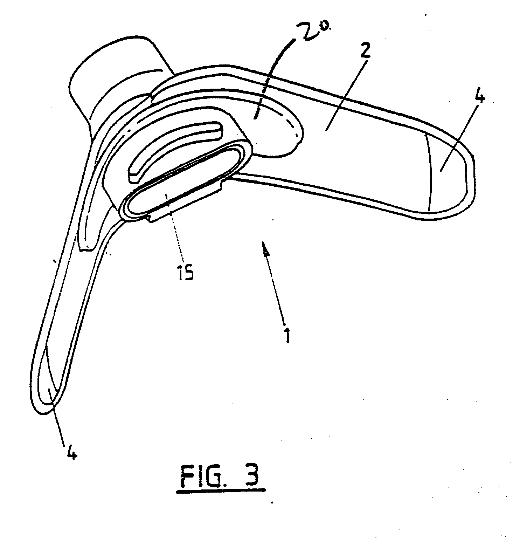

The present disclosure provides a system for oral delivery of gases pressurised above ambient to a user and is especially suited for use in the oral delivery of air in continuous positive airway pressure (CPAP) treatments of sleeping disorders such as sleep apnea. As shown in Figure 4, the system includes a mouthpiece 1 which is connected by a connection 40 to a breathing circuit 41.

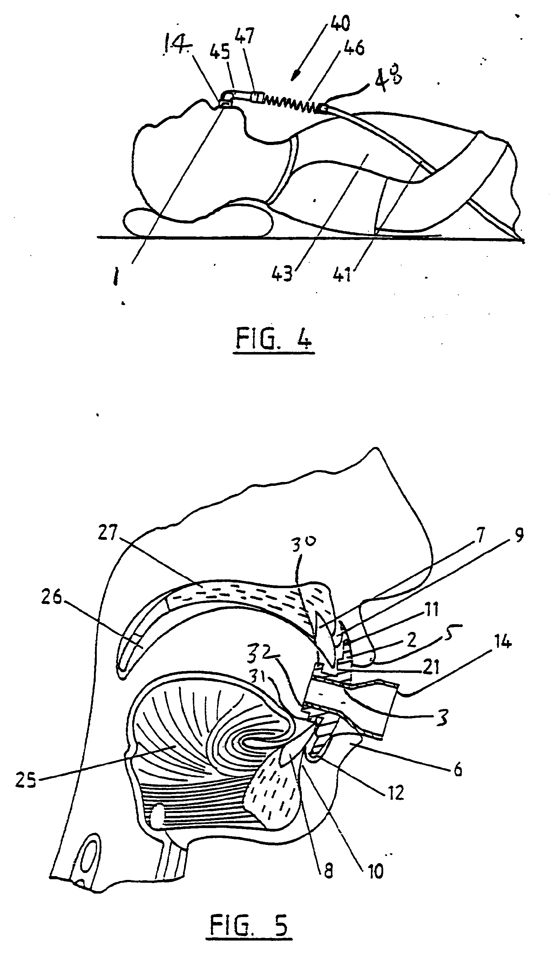

Attention is directed to Figures 1-3 and 5 which shows a first embodiment of the mouthpiece 1 of the present invention. The mouthpiece 1 is formed from a vestibular shield 2 which has a gases passage 3, see Figure 5, provided through a central portion 18 thereof. A gases inlet 14 is provided on a front or outer surface of the central portion 18 of the vestibular shield 2 and is aligned with the gases passageway 3 provided through the central portion 18 of the vestibular shield 2. A gases outlet 15 is provided on a rear or inner face of the central portion 18 of the vestibular shield 2 and is aligned with the gases passage 3 provided through the vestibular shield 2 and with the gases inlet 14.

With particular reference to Figures 1-3, the vestibular shield 2 is a generally flat and generally rectangularly-shaped member in front elevation having a curved profile that reflects the curvature of a user's jaw and in turn the curvature of the labial vestibule region (the region between the lips 5,6 and the front teeth 7,8 and the front gums 9,10). The vestibular shield 2 is dimensioned such that outer portions 4 extend from the central portion 18 and around the sides of the labial vestibule into the buccal vestibule region (the region between the back teeth and the cheeks). In addition, the outer portions 4 of the vestibular shield 2 have rounded upper and lower edges 13. As shown in Figure 5, the vestibular shield 2 has a vertical dimension such that the upper and lower edges 11, 12 extend beyond the margins of the teeth 7, 8 to overlap the gums 9, 10 in the labial vestibule region. The vertical dimension is generally consistent along the horizontal length of the vestibular shield 2 such that the upper and lower edges 11, 12 of the vestibular shield extend beyond the margins of the back teeth in the buccal vestibule region to overlap the gums of the back teeth.

The vertical and horizontal dimension of the vestibular shield 2 of the mouthpiece 1 is particularly significant in the CPAP application because the vestibular shield 2 provides a larger sealing area (in this case between the lips and the outer surface of the vestibular shield 2) than prior art oral devices. With the present mouthpiece 1, the vestibular shield 2 remains substantially sealed within the user's mouth even if the user is fully relaxed and the jaw becomes distended. Sealing of the mouthpiece 1 with the user's mouth is important because of the elevated pressure within the user's mouth during CPAP use. If an oral mouthpiece is not properly sealed within the user's mouth, which can occur with prior art mouthpieces, and sufficient air leaks result, the user can awaken. In addition, air leaks can result in a considerable drop in pressure which can cause recurring apnea. The shape and overall size of the present vestibular shield 2 in relation to a normal mouth opening size requires a conscious effort of lip manipulation by a user to configure the mouth opening for release of the mouthpiece 1 from the vestibule region.

The vestibular shield 2 of the present invention fits comfortably within the mouth of a user. It is preferred that at least the vestibular shield 2 of the mouthpiece 1 is made from a material that is supple and very soft so that the vestibular shield 2 adapts readily to the form of the user's mouth without significant fitting. Silicone rubber has been found to be an appropriate material. In addition the general shape of the vestibular shield 2 fits naturally within the mouth vestibule, although at a generic level. Each of the upper and lower edges 11,12 of the vestibular shield 2 is provided with notches 16,17 respectively at a center position of the vestibular shield 2 which accommodate the frenal attachments to the upper and lower lips. The rounded upper and lower edges 13 of the outer portions 4 conform substantially to the shape of a normal buccal vestibule. If the vestibular shield 2 is made from silicone rubber, it tends to be difficult to trim outer portions 4 of the vestibular shield 2, if required, but it has been found that excellent performance across a range of mouth sizes can be achieved with only a limited selection of vestibular shield 2 sizes.

Additionally, and having further benefits for user comfort, is the provision, see particularly Figures 2 and 3, of a raised area 20 on the inner face of the vestibular shield 2, such raised area 20 having a generally flattened oval shape 21 so that in use, see Figure 5, the raised area 20 abuts against the teeth 7,8 of a user and thereby reduces pressure and potential wear against the gums 9,10 of the user. Furthermore, this raised region 20 rigidifies the vestibular shield 2 without effectively increasing the overall thickness, as the raised region 20 lies within what would otherwise be a cavity, and allows the mouthpiece 1 to have a reasonable overall stiffness in bending within its curvature. This reduces the ability for the mouthpiece 1 to be removed accidentally as removal requires deformation in the curvature, while having very supple peripheral outer portions 4 which is important for comfort and sealing efficiency.

As shown in Figures 1-3 and 5, the gases inlet 14, gases passage 3 and the gases outlet 15 are in generally perpendicular alignment through the central portion 18 of the vestibular shield 2 and form a direct path through the vestibular shield 2. This configuration of the gases inlet 14, the gases passage 3 and the gases outlet 15 is preferred because of the simplicity of construction. This direct path gives less surface area and less flow impingement than more tortuous possible routes and therefore provides a slight reduction in pressure drop from the humidified gases stream passing through the passage during CPAP treatment.

The gases outlet 15 is formed from a stub tube or conduit and may be integrally formed with the vestibular shield 2, as shown in the drawings, or may be a separate member which is attached by suitable means to the vestibular shield 2. The gases outlet 15 is preferably of a generally flattened oval shape and includes a short flange 30 along its upper surface and a short flange 31 along its lower surface such that in use, see Figure 5, a users upper and lower teeth can rest on the gases outlet 15 in the space between the respective flanges 30, 31 and the vestibular shield 2. The flange 30 on the upper surface of the gases outlet 15 is preferably laterally short and adjacent the rear edge 32 of the gases outlet 15, while the flange 31 on the lower surface of the gases outlet 15 is preferably curved, more laterally extensive and set back from the rear edge 32 of the gases outlet 15 to promote retention of the lower jaw in a forward position which reduces airway resistance. Retention of the lower jaw in a forward position is a preferred position, but not a necessary one to effect the invention.

The gases inlet 14 is formed from a stub tube or conduit and may be integrally formed with the vestibular shield 2 or may be a separate member which is attached by suitable means to the vestibular shield 2. As shown in the drawings, the gases inlet 14 is formed from a conduit 34 that is inserted through the gases passage 3 and into the gases outlet 15. The inserted conduit 34 which forms the gases inlet 14 is preferably formed of a material which is significantly harder or stiffer than the surrounding supple material of which the vestibular shield 2 is formed. This provides a secure connection to the breathing circuit 41 and provides for durability of the mouthpiece 1. A moulded polyethylene tube may be used for the inserted conduit 34. The harder plastic material makes it possible to utilise standard breathing circuit connections at the gases inlet 14, and for this reason the inserted conduit 34 preferably merges from a generally flattened oval shape 35 at the outlet proximate to the exit of the gases outlet 15 to a circular shape 36 at the inlet. The inserted conduit 34 is preferably surrounded by the gases outlet 15 which is made of the more supple material, so that the surface 37 of the gases outlet 15 presented to the user is soft and pliable, while the hard plastic inserted conduit 34 ensures that the breathing passageway formed by the gases outlet 15 will not be accidentally collapsed by pressure from the jaws of the user. In use, the gases inlet 14 extends between the lips 5, 6 of the user.

One possible alternative configuration (not illustrated) of gases distribution through the mouthpiece 1 would be to route the gases from the centrally located gases inlet 14, through an internally located network of distribution passages to the outer portions 4 of the vestibular shield 2, whereupon the gases would exit though outlet apertures in the rear, or inner, surface of the vestibular shield 2. In this region, the apertures would be facing substantially inward towards the respective openings between the upper and lower jaw, behind the rearmost teeth when the jaw is closed. This has the possible advantage that the main air flows largely bypass the surface of the tongue 25 and the roof of the mouth (soft palate 26 and hard palate 27) and consequently, this configuration may show a reduction in mouth drying. In this configuration, it would of course be possible to leave off any semblance of the outlet conduit 15, allowing full jaw closure, although it is noted that slight jaw opening is a normal position for oral breathing. This alternative is not preferred as it increases the amount of material in the vestibular shield 2 thereby increasing stiffness of the vestibular shield 2 and reducing the effectiveness of the seal created by the vestibular shield 2. In addition, mouth drying is not an expected problem where the mouthpiece 1 is used with humidified gases. It is to be understood that other alternative paths of gases distribution through the mouthpiece 1 are within the scope of the present invention which may deliver other benefits.

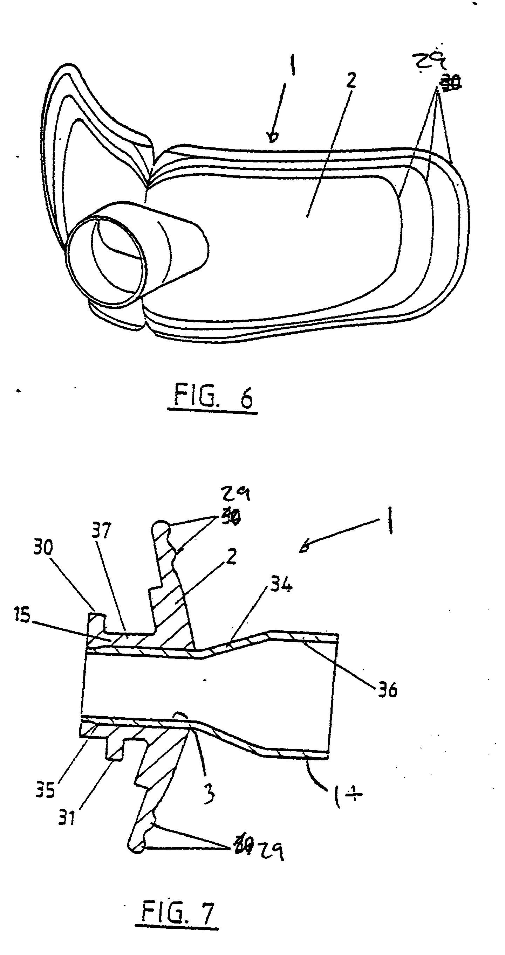

A variation of the present invention is illustrated in Figures 6 and 7. This variation is intended to reduce the number of different sized mouthpieces that are required to be produced to cover an entire size range. In particular, the vestibular shield 2 of the mouthpiece 1 is formed with a series of concentric ribs 29 at and following the periphery thereof. In use, a person fitting the mouthpiece 1 for a user will trim back the periphery of the vestibular shield 2, for example by cutting with a sharp knife or scissors, removing one or more of the ribs 29, using the valleys between the ribs 29 as a guide, as necessary for achieving a comfortable fit of the vestibular shield 2 within the user's vestibule. The ribs 29 serve primarily as a guide for trimming, but also the valleys provide a further element of suppleness to the periphery of the vestibular shield 2.

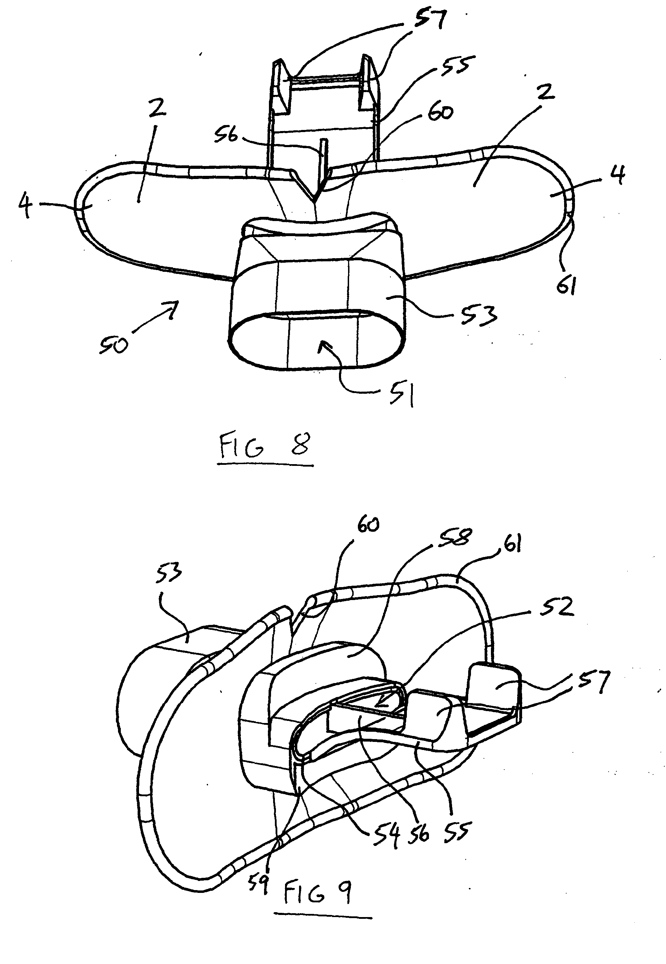

A further and preferred embodiment is illustrated in Figures 8 to 11. In this embodiment, the mouthpiece 50 includes a vestibular shield 2 substantially in accordance with the earlier embodiments. A gases passageway extends through the vestibular shield from an inlet 51 to an outlet 52 in much the same way as with the earlier embodiments. In the preferred embodiment 51 is provided by a flattened oval-shaped connector 53. The outlet 52 has an even more laterally extended flattened oval shape 54. The major differences between the mouthpiece 50 and the embodiments described above are provided on the inner face of the vestibular shield. Most prominently, the mouthpiece 50 includes a tongue depressor 55 extending from the inner face of the vestibular shield 2. The operation of the tongue depressor will be described further on with reference to Figure 11. The tongue depressor includes a vertical stiffening flange 56 centrally located on its upper surface and extending from the gases outlet 52. In use gases flow easily around the stiffening flange 56 effectively bifurcating the gases outlet 52. The tongue depressor 55 further includes a pair of vertically extending spacers 57 which in use may abut against the roof of the wearer's mouth and ensure that the tongue cannot completely block the air passageway. In the mouthpiece 50 the sealing effect of the vestibular shield 2 against the lips of the user is enhanced by providing teeth abutments of significantly increased thickness than the raised area 20 of the earlier embodiments. In particular, an upper teeth abutment 58 and a lower teeth abutment 59 are provided, with the lower teeth abutment 59 protruding further from the inner face of the vestibular shield 2 than the upper teeth abutment 58. This difference serves to match the typical over-bite of most users. The abutments 58 and 59 are not required to be wider than the gases outlet 52.

A notch 60 is provided centrally in the upper edge of the vestibular shield 2 to accommodate the upper frenal attachment. A slight bead 61 is provided around the edge of the vestibular shield 2 for user comfort, with the vestibular shield 2 otherwise being very thin for additional suppleness.

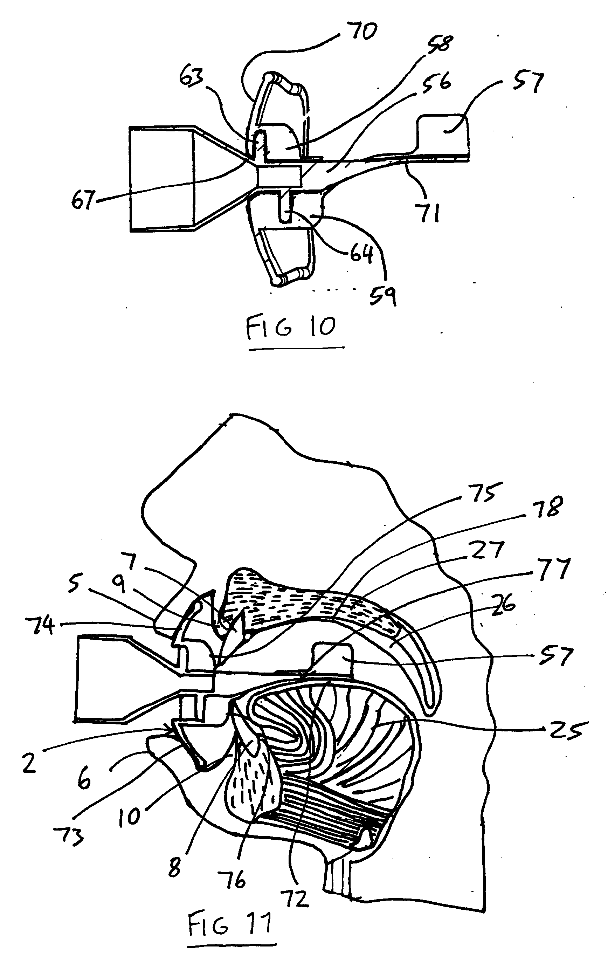

Referring particularly to Figure 10, in its preferred form the mouthpiece 50 is preferably formed by over-moulding a soft and supple material part 70 over a stiffer material part 67. These can generally be termed the shield part and the passageway-forming insert. The passageway-forming insert preferably includes a pair of upper and lower vertical flanges 63 and 64 to fully engage within the supple material. The passageway-forming insert 67 includes the vertically extending stiffening flange 56 of the tongue depressor 55, together with a curved planar portion 71 forming the backbone of the tongue depressor 55. The vertically extending spacers 57 are of the soft and supple material and are part of the over-moulding 70, as are the upper and lower teeth abutments 58 and 59.

Referring now to Figure 11, use of the mouthpiece according to Figures 8 to 10 is depicted. Reference numerals concerning the anatomical features of the user are common with those numerals used in Figure 5. With the present mouthpiece 50, the upper and lower lips 5, 6 are further distended by the abutment action of the abutments 75, 76 against the upper and lower teeth 7, 8 respectively, thus forming a seal of greater pressure between the lips 5, 6 and the upper and lower portions respectively of the vestibular shield 2. A lower face 77 of the tongue depressor 55 impinges if necessary on the upper surface 72 of the tongue 25 and retains the tongue in the lower portion of the mouth. This ensures a clear gases outlet 52 from the gases passageway through the vestibular shield. The vertically extending spacers 57, if forced by pressure from the tongue, will engage against the roof of the user's mouth and maintain a clear air passageway. This stops the sleeping patient unconsciously blocking the oral passageway and reverting to nasal breathing.

Attention is now directed to Figure 4. It has been found that an additional factor in the effectiveness of any mouthpiece, including mouthpiece 1, is the manner in which the mouthpiece is connected to the breathing circuit 41. The weight of the breathing circuit 41, and any attempted movement of one other of the breathing circuit 41 and the mouthpiece 1 relative to the other, is one of the largest influences tending to dislodge a mouthpiece 1 from the mouth of a user. It must be noted that the mouthpiece 1 must remain in position and maintain a seal during REM sleep, when the user has no muscle tone.

The connection 40 as provided in the present invention between the breathing circuit 41 and the mouthpiece 1 decouples the mouthpiece 1 from the breathing circuit 41. As a result, the connection 40 is effective in reducing the forces placed on the mouthpiece 1 by the breathing circuit 41 when the user moves around during sleep. In the preferred sleeping position, the breathing circuit 41 is laid across the chest 43 of the user, and may be secured to the user's bed clothes or sleeping garments. The breathing circuit 41 is preferably laid on the chest of the user to take the weight of the breathing circuit 41 off of the mouthpiece 1.

To connect between the gases outlet 14 which is vertical when the user is laying on his or her back and the breathing circuit 41 which is generally horizontal, an L-shaped elbow 45 is incorporated in the connection 40. The elbow 45 may be incorporated in the mouthpiece 1, however, it is preferred that the mouthpiece 1 be kept small to provide for easier cleaning. The elbow 45 is formed at a right angle and provides a positive pressure on the mouthpiece 1 to maintain the mouthpiece 1 in the user's mouth. The elbow 45 may include a swivel joint. The connection 40 further includes an extremely flexible connecting tube 46 provided between the elbow 45 and the breathing circuit 41. The connecting tube 46 is preferably connected to the breathing circuit 41 by a swivel joint 48 for reasons described herein. The breathing circuit 41, while flexible, will necessarily be stiff enough to maintain its integrity over comparatively long tuns, while the connecting tube 46, being only a short length, for example 10 centimeters, merely has to span between the user's mouth and chest, and can thereby be made in a manner that would not be suitable for long runs. Furthermore, as a result of the short length of the connecting tube 46, the connecting tube 46 does not need to incorporate significant insulation or heating capability. The connecting tube 46 may be formed from a thin plastic membrane supported over a helical or double helical supporting ribs. In such a case, the support makes the connection tube 46 laterally flexible and resistant to torsion. The elbow swivel joint 45 allows for movement of the connection tube 46 relative to the mouthpiece 1. The swivel joint 48 allows for movement of the connection tube 46 relative to the breathing circuit 41. It is to be understood that one or both of the swivel joints 45, 48 could be eliminated, but the preferred embodiment includes swivel joint 48.

From the above it can be seen that the present invention provides a system including mouthpiece 1 for oral delivery of CPAP treatment which at once is low cost and effective. Unlike other appliances the mouthpiece 1 used in the present invention does not require custom orthodontic fitting as the mouthpiece 1 does not rely on accurate alignment with the user's teeth or the user's palate to provide location and retention within the user's mouth, but instead resides in the vestibule between the teeth and lips and the teeth and cheeks, and the lateral and vertical extension of the vestibular shield 2 requires that the user's lips be actively manipulated for the vestibular shield 2 to be removed. Furthermore the improved connection 40 to the breathing circuit 41 reduces the forces which tend to pull at the mouthpiece 1.

While the preferred embodiments of the present invention are shown and described, it is envisioned that those skilled in the art may devise various modifications of the present invention without departing from the spirit and scope of the appended claims.

The features disclosed in the foregoing description, in the claims and/or in the accompanying drawings may, both separately and in any combination thereof, be material for realising the invention in diverse forms thereof.

## Claims

1. A mouthpiece for delivery of gases to the oral cavity of a user comprising a generally rectangularly-shaped vestibular shield (2) having an inner surface and an outer surface, and gases passageway means (3) extending from said outer surface of said vestibular shield (2) to said inner surface of said vestibular shield (2) for allowing the passage of gases through said mouthpiece (1), said vestibular shield (2) having a predetermined height which will overlap a user's teeth and gums when positioned in the mouth vestibule of a user, said vestibular shield (2) having a central portion (18) that will extend over a user's front teeth and gums when said central portion (18) of said vestibular shield (2) is positioned between the lips and the teeth of the user **characterised in that** said vestibular shield, has outer portions (4) extending from said central portion (18) which extend along and overlap at least a portion of the user's back teeth and gums when said outer portions (4) of said vestibular shield (2) are positioned between the cheeks and the teeth of the user, said vestibular shield (2) in use sealing against the inner walls of the lips and/or cheeks of said user's mouth.

2. A mouthpiece as claimed in claim 1, further including a raised portion provided on said inner surface of said central portion (18) of said vestibular shield (2), said raised portion solely contacting the teeth of a user when said vestibular shield (2) is placed in the mouth of a user.

3. A mouthpiece as claimed in claim 2 wherein said raised portion (20) is of a generally flattened oval shape.

4. A mouthpiece as claimed in either claim 2 or claim 3, wherein said raised portion (20) extends along at least a portion of said outer portions (4)

5. A mouthpiece as claimed in any one of claims 1 to 4, wherein said central portion (18) includes a centrally located notch (16) in a top edge (11) thereof

6. A mouthpiece as claimed in claim 5, further including a centrally located notch (17) in a bottom edge (12) of said central portion (18).

7. A mouthpiece as claimed in any one of claims 1 to 6, wherein said vestibular shield (2) has generally curved profile.

8. A mouthpiece as claimed in any one of claims 1 to 7, wherein said gases passageway means (3) includes gases inlet means (14) for allowing connecting of said mouthpiece (1) to a gases supply.

9. A mouthpiece as claimed in claim 8, wherein said gases inlet means (14) is connected to said central portion (18) of said vestibular shield (2).

10. A mouthpiece as defined in claim 9, wherein said vestibular shield (2) is formed of a supple material and said gases inlet means (14) comprises a tube (34) formed of a stiffer material than said supple material that is used to form said vestibular shield (2), said tube (34) being attached to said vestibular shield (2).

11. A mouthpiece as claimed in any one of claims 1 to 10, wherein said gases passageway means (3) includes gases outlet means (15) for allowing gases to be expelled into the mouth of a user.

12. A mouthpiece as claimed in claim 11, wherein said gases outlet means (15) ism provided along said central portion (18) of said vestibular shield (2).

13. A mouthpiece as claimed in claim 11, wherein said gases passageway means (3) includes gases inlet means (14) for allowing connection of said mouthpiece (1) to a gases supply

14. A mouthpiece as claimed in claim 13 wherein said gases outlet means (15) is aligned with said gases inlet means (14).

15. A mouthpiece as claimed in claim 14, wherein said gases inlet means (14) and said gases outlet means (15) are connected to said central portion (18) of said vestibular shield (2).

16. A mouthpiece as claimed in claim 11, wherein said gases outlet means (15) is a generally flattened oval-shaped tube (34) which extends from said inner surface of said vestibular shield (2).

17. A mouthpiece as claimed in claim 16, wherein said generally flattened oval-shaped tube (34) has an upper surface and a lower surface, and said gases outlet means (15) further includes a flange along at least one of said upper and lower surfaces, said flange being spaced from said vestibular shield (2).

18. A mouthpiece as claimed in claim 16, wherein said generally flattened oval-shaped tube (34) has an upper surface and a lower surface, and said gases outlet means (15) further includes an upper flange (30) extending along said upper surface, said upper flange (30) being spaced from said vestibular shield (2), and a lower flange (31) extending along said lower surface, said lower flange (31) being spaced from said vestibular shield (2).

19. A mouthpiece as claimed in claim 18, wherein said upper flange (30) is adjacent a rear end of said gases outlet means (15) and said lower flange (31) is spaced from said rear end of said gases outlet means (15), said lower flange being curved.

20. A mouthpiece as claimed in any one of claims 1 to 19, wherein said outer portions (4) of said vestibular shield (2) include a plurality of ribs (30) thereon.

21. A mouthpiece as claimed in claim 20, wherein said ribs (30) are provided on said outer surface of said vestibular shield (2).

22. A mouthpiece as claimed in any one of claims 1 to 21, wherein said vestibular shield (2) is formed of a supple material.

23. mouthpiece as claimed in any one of claims 1 to 22, wherein said vestibular shield (2) is generally flat.

24. A mouthpiece as claimed in any one of claims 1 to 23, wherein each said outer portion (4) has rounded upper and lower edges (13).

25. A mouthpiece as claimed in either claim 2 or claim 3 wherein said raised portion includes an upper abutment portion (58) for abutting against the upper teeth and a lower abutment portion (59) for abutting against the lower teeth and the lower abutment portion (59) is raised further from the inner surface of the vestibular shield (2) than said upper abutment portion (58).

26. A mouthpiece as claimed in any one of claims 1 to 25 including a tongue depressing guard (55) extending from the inner side of said vestibular shield (2)

27. A mouthpiece as claimed in claim 26 wherein said tongue depressing guard (55) has a concave curve in its lower surface and is stiffer than the outer portions (4) of said vestibular shield.

28. A mouthpiece as claimed in either claim 26 or claim 27 including one or more vertically extending spacers (57) projecting from the upper surface of said tongue depressing guard (55).

29. A mouthpiece as claimed in any one of claims 26 to 28 wherein said gases passageway (3) is provided by an insert (67) of substantially stiffer material than the material of said vestibular shield (2), said insert (67) extending through said vestibular shield (2), and said tongue depressing guard (55) includes therein an extension of said insert (67) forming a stiff backbone (71) therefor.

## Patentansprüche

1. Mundstück zum Zuführen von Gasen in die Mundhöhle eines Verwenders, welches einen im Allgemeinen rechteckförmigen Vorhofschild (2) umfasst, der eine innere Fläche und eine äußere Fläche aufweist, und Gasdurchgangsmittel (3), die sich von der äußeren Fläche des Vorhofschilds (2) zu der inneren Fläche des Vorhofschilds (2) erstrecken, um den Durchtritt von Gasen durch das Mundstück (1) zu erlauben, wobei der Vorhofschild (2) eine vorbestimmte Höhe aufweist, welche die Zähne und das Zahnfleisch eines Verwenders überlappt, wenn dieser im Mundvorhof eines Verwenders angeordnet ist, wobei der Vorhofschild (2) einen Mittelabschnitt (18) aufweist, der sich über die vorderen Zähne und das vordere Zahnfleisch erstreckt, wenn der Mittelabschnitt (18) des Vorhofschilds (2) zwischen den Lippen und den Zähnen des Verwenders angeordnet ist,
**dadurch gekennzeichnet, dass** der Vorhofschild vom Mittelabschnitt (18) ausgehende, äußere Abschnitte (4) aufweist, die sich entlang zumindest eines Abschnitts der hinteren Zähne und des hinteren Zahnfleischs des Verwenders erstrecken und diesen überlappen, wenn die äußeren Abschnitte (4) des Vorhofschilds (2) zwischen den Wangen und den Zähnen des Verwenders angeordnet sind; wobei der Vorhofschild (2) sich im Gebrauch dicht gegen die inneren Wände der Lippen und/oder der Wangen des Munds des Verwenders anlegt.

2. Mundstück gemäß Anspruch 1, welches ferner einen auf der inneren Fläche des Mittelabschnitts (18) des Vorhofschilds (2) vorgesehenen, erhöhten Abschnitt einschließt, wobei der erhöhte Abschnitt dann, wenn der Vorhofschild (2) im Mund eines Verwenders angeordnet ist, ausschließlich mit den Zähnen eines Verwenders in Kontakt tritt.

3. Mundstück gemäß Anspruch 2, bei welchem der erhöhte Abschnitt (20) eine im Allgemeinen abgeflachte, ovale Gestalt aufweist.

4. Mundstück gemäß Anspruch 2 oder Anspruch 3, bei welchem sich der erhöhte Abschnitt (20) entlang zumindest eines Abschnitts der äußeren Abschnitte (4) erstreckt.

5. Mundstück gemäß einem der Ansprüche 1 bis 4, bei welchem der Mittelabschnitt (18) in seinem oberen Rand (11) eine zentral gelegene Kerbe (16) einschließt.

6. Mundstück gemäß Anspruch 5, welches ferner in einem unteren Rand (12) des Mittelabschnitts (18) eine zentral gelegene Kerbe (17) einschließt.

7. Mundstück gemäß einem der Ansprüche 1 bis 6, bei welchem der Vorhofschild (2) ein im Allgemeinen gebogenes Profil aufweist.

8. Mundstück gemäß einem der Ansprüche 1 bis 7, bei welchem die Gasdurchgangsmittel (3) Gaseinlassmittel (14) einschließen, um das Verbinden des Mundstücks (1) mit einer Gaszuführung zu erlauben.

9. Mundstück gemäß Anspruch 8, bei welchem die Gaseinlassmittel (14) mit dem Mittelabschnitt (18) des Vorhofschilds (2) verbunden sind.

10. Mundstück gemäß Anspruch 9, bei welchem der Vorhofschild (2) aus einem biegsamen Material gebildet ist und die Gaseinlassmittel (14) ein Rohr (34) umfassen, welches aus einem steiferen Material gebildet ist als das biegsame Material, welches zum Bilden des Vorhofschilds (2) verwendet wird, wobei das Rohr (34) am Vorhofschild (2) befestigt ist.

11. Mundstück gemäß einem der Ansprüche 1 bis 10, bei welchem die Gasdurchgangsmittel (3) Gasauslassmittel (15) einschließen, um das Ausstoßen von Gasen in den Mund eines Verwenders zu erlauben.

12. Mundstück gemäß Anspruch 11, bei welchem die Gasauslassmittel (15) entlang des Mittelabschnitts (18) des Vorhofschilds (2) vorgesehen sind.

13. Mundstück gemäß Anspruch 11, bei welchem die Gasdurchgangsmittel (3) Gaseinlassmittel (14) einschließen, um das Verbinden des Mundstücks (1) mit einer Gaszuführung zu erlauben.

14. Mundstück gemäß Anspruch 13, bei welchem die Gasauslassmittel (15) zu den Gaseinlassmitteln (14) ausgerichtet sind.

15. Mundstück gemäß Anspruch 14, bei welchem die Gaseinlassmittel (14) und die Gasauslassmittel (15) mit dem Mittelabschnitt (18) des Vorhofschilds (2) verbunden sind.

16. Mundstück gemäß Anspruch 11, bei welchem die Gasauslassmittel (15) ein im Allgemeinen abgeflachtes, ovalförmiges Rohr (34) sind, welches von der inneren Fläche des Vorhofschilds (2) ausgeht.

17. Mundstück gemäß Anspruch 16, bei welchem das im Allgemeinen abgeflachte, ovalförmige Rohr (34) eine obere Fläche und eine untere Fläche aufweist, und die Gasauslassmittel (15) entlang der oberen und/oder der unteren Fläche ferner einen Flansch einschließen, wobei der Flansch vom Vorhofschild (2) beabstandet ist.

18. Mundstück gemäß Anspruch 16, bei welchem das im Allgemeinen abgeflachte, ovalförmige Rohr (34) eine obere Fläche und eine untere Fläche aufweist, und die Gasauslassmittel (15) ferner einen sich entlang der oberen Fläche erstreckenden, oberen Flansch (30) einschließen, wobei der obere Flansch (30) vom Vorhofschild (2) beabstandet ist, sowie einen sich entlang der unteren Fläche erstreckenden, unteren Flansch (31), wobei der untere Flansch (31) vom Vorhofschild (2) beabstandet ist.

19. Mundstück gemäß Anspruch 18, bei welchem sich der obere Flansch (30) in Nachbarschaft eines hinteren Endes der Gasauslassmittel (15) befindet und der untere Flansch (31) vom hinteren Ende der Gasauslassmittel (15) beabstandet ist, wobei der untere Flansch gebogen ist.

20. Mundstück gemäß einem der Ansprüche 1 bis 19, bei welchem die äußeren Abschnitte (4) des Vorhofschilds (2) auf sich eine Mehrzahl von Rippen (30) einschließen.

21. Mundstück gemäß Anspruch 20, bei welchem die Rippen (30) auf der äußeren Fläche des Vorhofschilds (2) vorgesehen sind.

22. Mundstück gemäß einem der Ansprüche 1 bis 21, bei welchem der Vorhofschild (2) aus einem biegsamen Material gebildet ist.

23. Mundstück gemäß einem der Ansprüche 1 bis 22, bei welchem der Vorhofschild (2) im Allgemeinen flach ist.

24. Mundstück gemäß einem der Ansprüche 1 bis 23, bei welchem jeder äußere Abschnitt (4) gerundete obere und untere Ränder (13) aufweist.

25. Mundstück gemäß Anspruch 2 oder Anspruch 3, bei welchem der erhöhte Abschnitt einen oberen Anlageabschnitt (58) einschließt, um sich gegen die oberen Zähne anzulegen, sowie einen unteren Anlageabschnitt (59), um sich gegen die unteren Zähne anzulegen, und der untere Anlageabschnitt (59) weiter über die innere Fläche des Vorhofschilds (2) erhöht ist als der untere Anlageabschnitt (58).

26. Mundstück gemäß einem der Ansprüche 1 bis 25, welches eine Zungenniederdrücksicherung (55) einschließt, die von der Innenseite des Vorhofschilds (2) ausgeht.

27. Mundstück gemäß Anspruch 26, bei welchem die Zungenniederdrücksicherung (55) in ihrer unteren Fläche eine konkave Biegung aufweist und steifer ist als die äußeren Abschnitte (4) des Vorhofschilds.

28. Mundstück gemäß Anspruch 26 oder Anspruch 27, welches einen oder mehrere sich vertikal erstreckende Abstandhalter (57) einschließt, die von der oberen Fläche der Zungenniederdrücksicherung (55) vorspringen.

29. Mundstück gemäß einem der Ansprüche 26 bis 28, bei welchem der Gasdurchgang (3) durch einen Einsatz (67) vorgesehen ist, der aus im Wesentlichen steiferem Material gebildet ist als der Vorhofschild (2), wobei der Einsatz (67) sich durch den Vorhofschild (2) erstreckt, und die Zungenniederdrücksicherung (55) in sich eine Erweiterung des Einsatzes (67) einschließt, die hierfür eine Versteifung (71) bildet.

## Revendications

1. Embouchure destinée à délivrer des gaz à la cavité orale d'un utilisateur, laquelle embouchure comprend un écran vestibulaire (2) de forme générale rectangulaire présentant une surface intérieure et une surface extérieure, et un moyen formant passage de gaz (3) s'étendant de ladite surface extérieure dudit écran vestibulaire (2) vers ladite surface intérieure dudit écran vestibulaire (2) pour permettre le passage de gaz à travers ladite embouchure (1), ledit écran vestibulaire (2) ayant une hauteur prédéterminée qui couvre les dents et les gencives d'un utilisateur lorsqu'il est positionné dans le vestibule de la bouche d'un utilisateur, ledit écran vestibulaire (2) comportant une portion centrale (18) qui s'étend par-dessus les dents et les gencives avant d'un utilisateur lorsque ladite portion centrale (18) dudit écran vestibulaire (2) est positionnée entre les lèvres et les dents de l'utilisateur, **caractérisée en ce que** ledit écran vestibulaire comporte des portions extérieures (4) s'étendant de ladite portion centrale (18) qui s'étend le long d'au moins une portion des dents et des gencives arrière de l'utilisateur en les recouvrant lorsque lesdites portions extérieures (4) dudit écran vestibulaire (2) sont positionnées entre les joues et les dents de l'utilisateur, ledit écran vestibulaire (2) faisant étanchéité en utilisation contre les parois intérieures des lèvres et/ou des joues de ladite bouche de l'utilisateur.

2. Embouchure selon la revendication 1, comprenant en outre une portion surélevée placée sur ladite surface intérieure de ladite portion centrale (18) dudit écran vestibulaire (2), ladite portion surélevée étant seulement en contact avec les dents d'un utilisateur lorsque ledit écran vestibulaire (2) est placé dans la bouche d'un utilisateur.

3. Embouchure selon la revendication 2, dans laquelle ladite portion surélevée (20) à une forme générale ovale aplatie.

4. Embouchure selon la revendication 2 ou 3, dans laquelle ladite portion en surélevée (20) s'étend le long d'au moins une portion desdites portions extérieures (4).

5. Embouchure selon l'une quelconque des revendications 1 à 4, dans laquelle ladite portion centrale (18) comprend une encoche (16) ménagée au centre dans un bord supérieur (11) de ladite portion centrale.

6. Embouchure selon la revendication 5, comprenant en outre une encoche (17) ménagée au centre dans un bord inférieur (12) de ladite portion centrale (18).

7. Embouchure selon l'une quelconque des revendications 1 à 6, dans laquelle ledit écran vestibulaire (2) a un profil général incurvé.

8. Embouchure selon l'une quelconque des revendications 1 à 7, dans laquelle ledit moyen formant passage de gaz (3) comprend un moyen d'entrée de gaz (14) permettant le raccordement de ladite embouchure (1) à une alimentation en gaz.

9. Embouchure selon la revendication 8, dans laquelle ledit moyen d'entrée de gaz (14) est raccordé à ladite portion centrale (18) dudit écran vestibulaire (2).

10. Embouchure selon la revendication 9, dans laquelle ledit écran vestibulaire (2) est formé à partir d'un matériau souple et ledit moyen d'entrée de gaz (14) comprend un tube (34) formé à partir d'un matériau plus rigide que le matériau souple qui est utilisé pour former ledit écran vestibulaire (2), ledit tube (34) étant fixé audit écran vestibulaire (2).

11. Embouchure selon l'une quelconque des revendications 1 à 10, dans laquelle ledit moyen de passage de gaz (3) comprend un moyen de sortie de gaz (15) permettant d'évacuer les gaz dans la bouche d'un utilisateur.

12. Embouchure selon la revendication 11, dans laquelle ledit moyen de sortie de gaz (15) est placé le long de ladite portion centrale (18) dudit écran vestibulaire (2).

13. Embouchure selon la revendication 11, dans laquelle ledit moyen de passage de gaz (3) comprend un moyen d'entrée de gaz (14) permettant de raccorder ladite embouchure (1) à une alimentation en gaz.

14. Embouchure selon la revendication 13, dans laquelle ledit moyen de sortie de gaz (15) est aligné avec ledit moyen d'entrée de gaz (14).

15. Embouchure selon la revendication 14, dans laquelle ledit moyen d'entrée de gaz (14) et ledit moyen de sortie de gaz (15) sont raccordés à ladite portion centrale (18) dudit écran vestibulaire (2).

16. Embouchure selon la revendication 11, dans laquelle ledit moyen de sortie de gaz (15) est un tube (34) de forme générale ovale aplatie qui s'étend depuis ladite surface intérieure dudit écran vestibulaire (2).

17. Embouchure selon la revendication 16, dans laquelle ledit tube (34) de forme générale ovale aplatie présente une surface supérieure et une surface inférieure, et ledit moyen de sortie de gaz (15) inclut en outre une nervure le long d'au moins une desdites surfaces supérieure et inférieure, ladite nervure étant espacée dudit écran vestibulaire (2).

18. Embouchure selon la revendication 16, dans laquelle ledit tube (34) de forme générale ovale aplatie présente une surface supérieure et une surface inférieure, et ledit moyen de sortie de gaz (15) inclut en outre une nervure supérieure (30) s'étendant le long de ladite surface supérieure, ladite nervure supérieure (30) étant espacée dudit écran vestibulaire (2), et une nervure inférieure (31) s'étendant le long de ladite surface inférieure, ladite nervure inférieure (31) étant espacée dudit écran vestibulaire (2).

19. Embouchure selon la revendication 18, dans laquelle ladite nervure supérieure (30) est proche d'une extrémité arrière dudit moyen de sortie de gaz (15) et ladite nervure inférieure (31) est espacée de ladite extrémité arrière dudit moyen de sortie de gaz (15), ladite nervure inférieure étant incurvée.

20. Embouchure selon l'une quelconque des revendications 1 à 19, dans laquelle lesdites portions extérieures (4) dudit écran vestibulaire (2) comprennent une pluralité de nervures (30).

21. Embouchure selon la revendication 20, dans laquelle lesdites nervures (30) sont placées sur ladite surface extérieure dudit écran vestibulaire (2).

22. Embouchure selon l'une quelconque des revendications 1 à 21, dans laquelle ledit écran vestibulaire (2) est formé à partir d'un matériau souple.

23. Embouchure selon l'une quelconque des revendications 1 à 22, dans laquelle ledit écran vestibulaire (2) est généralement plat

24. Embouchure selon l'une quelconque des revendications 1 à 23, dans laquelle chaque portion extérieure (4) comporte des bords arrondis supérieur et inférieur (13).

25. Embouchure selon la revendication 2 ou la revendication 3, dans laquelle ladite portion surélevée inclut une portion de butée supérieure (58) destinée à buter contre les dents supérieures et une portion de butée inférieure (59) destinée à buter contre les dents inférieures, la portion de butée inférieure (59) étant davantage surélevée de la surface intérieure de l'écran vestibulaire (2) que ladite portion de butée supérieure (58).

26. Embouchure selon l'une quelconque des revendications 1 à 25 incluant une garde d'abaissement de langue (55) s'étendant depuis le côté intérieur dudit écran vestibulaire (2).

27. Embouchure selon la revendication 26, dans laquelle ladite garde d'abaissement de langue (55) présente une courbure concave dans sa surface inférieure et est plus rigide que les portions extérieures (4) dudit écran vestibulaire.

28. Embouchure selon la revendication 26 ou 27 comprenant une ou plusieurs entretoises (57)¹ s'étendant verticalement en saillie depuis la surface supérieure de ladite garde d'abaissement de langue (55).

29. Embouchure selon l'une des revendications 26 à 28, dans laquelle ledit passage de gaz (3) est réalisé par un insert (67) en matériau sensiblement plus rigide que le matériau dudit écran vestibulaire (2), ledit insert (67) s'étendant à travers ledit écran vestibulaire (2), et dans laquelle ladite garde d'abaissement de langue (55) inclut une extension dudit insert (67) formant une ossature rigide (71) pour ladite garde d'abaissement de langue.
